# EUROPEAN PATENT SPECIFICATION

(11) **EP 4 325 051 B1**
(45) Date of publication and mention of the grant of the patent: **17.12.2025**
(21) Application number: 22190844.5
(22) Date of filing: 17.08.2022
(51) Int. Cl.: A61M 5/142, F04B 13/00, F04B 23/06, F04B 53/10, F04B 53/14, F04B 53/16

(54) **SINGLE USE VOLUMETRIC DOSAGE PUMP KIT**
VOLUMETRISCHES EINWEG-DOSIERPUMPENKIT
KIT DE POMPE DE DOSAGE VOLUMÉTRIQUE À USAGE UNIQUE

(43) Date of publication of application: 21.02.2024
(73) Proprietor: Neoceram S.A., 7110 Strepy-Bracquegnies (BE)
(72) Inventor: MOREL, Alexandre, Pittsburgh, 15202 (US); SARWA, Andre, 69340 Francheville (FR); LEDEGANCK, Robin, 7160 Piéton (BE)
(74) Representative: Kirkpatrick

(56) References cited:
- EP-A1- 2 140 892
- WO-A1-2016/012567
- DE-A1- 10 305 441
- GB-A- 2 431 439

## Description

The invention relates to the field of volumetric pumps and in particular single use pumps for use in sterile environments. In particular the present invention relates to a set comprising a kit and a driving system adapted to operate the kit, said kit comprising:
- a pump with a piston slidingly arranged within a cylinder body, the cylinder body being arranged with an inlet and an outlet both allowing fluid communication between the outside of the cylinder and a metering chamber inside of the cylinder body, said metering chamber being further defined by one extremity of the piston lying within the cylinder body and wherein the other extremity of the piston is protruding from the cylinder body;
- an inlet connecting line fastened to the inlet;
- an outlet connecting line fastened to the outlet;

wherein the connecting lines are made with a flexible material allowing the line to be obturated upon pinching;
said driving system comprising pinching means arranged for pinching the outlet connecting line and for managing a fluid circulation in the outlet connecting line, said driving system further comprising a mobile arm (51) arranged to hold said other extremity of the piston and for driving a reciprocal translation of said piston within the cylinder body.

Such a set is known from EP 2 140 892. In the known set the pump is driven in a reciprocal translation movement within the cylinder. The known set also foresees the possibility to apply pinching means on the outlet connecting line in order to avoid inadvertent delivery of fluid.

A drawback of the known set is that the pump is provided with valves on the inlet and outlet. Such valves can lead to leaks and therefor the pinching means are applied on the outlet.

It is an object of the present invention to provide a set comprising a kit and a driving system enabling to operate by alternatively hindering a fluid displacement in the inlet and outlet connecting line without having to use check valves or non-return valves.

To this purpose a set according to the present invention is characterized in that the pinching means are further provided for pinching the inlet connecting line and for managing a fluid circulation in the inlet connecting line, said pinching means being further arranged to alternatively pinch the inlet connecting line and the outlet connecting line, said mobile arm and said pinching means being synchronized so as to close the output connecting line when the piston is translated outwards of the cylinder body and to close the input connecting line when the piston is translated inwards of the cylinder body.

It should further be noted that GB 2 431 439 also describes a set comprising a kit and a driving system. In the latter set there is however no teaching to synchronize the mobile arm and the pinching means.

Industries like the pharmaceutical industry or the medical industry use volumetric pumps, or positive displacement pumps, to fill individual containers with liquid preparations, like for example pharmaceutical preparations into syringes (vaccines), solutions into pouches for intravenous administrations, etc. ... It is of utmost importance that the liquid preparations remain free of foreign particulates and sterile and that the volume dispensed is very precise, even with very small volumes below 1 mL or below 0.1 mL.

To reach these objectives, high precision volumetric pumps have been developed, in various materials, like stainless steel or even ceramics. Ceramics are widely used by pharmaceutical producers to remove risk of contamination from chromium often associated to metal pumps.

These pumps are usually built around a main central axis defined by a cylinder, hollowed out in a casing, in which a piston is slidingly longitudinally moveable, with a predefined amplitude to define a metering chamber having a determined volume. We also refer to such systems as piston pumps. In some cases, referred to as two-element piston pumps, a channel is arranged in the piston which additionally rotates around the axis to alternatively connect the metering chamber with the inlet and the outlet nozzles of the pump, said channel therefore serving as a switching valve. In other cases, referred to as three-element pumps, the switching valve is built in a separate element, also inserted in the cylinder. In this later case, the piston is actioned via a sliding element while the switching valve is actioned with a separate rotating element. The person skilled in the art is familiar with these pumps and EP3045724 discloses how such pumps work.

An inlet is connected to a bulk tank of the liquid to distribute, via tubings and an outlet is usually connected, via tubings, to a needle for end distribution in the final container.

Volumetric pumps need to be regularly cleaned and sterilized, for example when starting a new production batch, to avoid contaminations from a variety of particles, cross contamination (i.e. contamination between pharmaceutical preparations of different compositions) or bacteriological growth.

For every product and used filling equipment in direct contact with such product, a cleaning procedure must be developed, performed and validated for their compliance by the pharmaceutical manufacturer. This implies that a cleaning validation dossier is put in place and tests must be performed to prove that the defined cleaning method leads to the required equipment cleanness and sterility, hence providing safety to patients. Such validation processes typically take several months and must be performed again when the filling equipment or the product is changed. This allows very little flexibility and places an important pressure on manufacturers as it significantly lengthens the time to market of a product.

To overcome this problem, single use pumps have been developed and used for a long time by manufacturers. These allow to switch the validation burden onto the pump manufacturer instead of the final product manufacturer. For many years, the most common solution has been the peristaltic pump, widely used as a single use alternative to volumetric pumps by pharmaceutical manufacturers.

The challenge for single use pumps is to combine high accuracy for small volumes (i.e. volumes lower than 0.3 ml) in scale-up manufacturing conditions, compatibility with viscous products, compatibility with biopharmaceutical products (i.e. protein-based products) populating the growing gene therapy market and quality of product after filling operations. Other challenges involve their installation in isolators while maintaining aseptic practices.

The applicant therefore judges necessary to propose a new single use volumetric pump meeting the stringent requirements for compliance in the pharmaceutical and/or biomedical industry.

Preferably, the extremity of the piston protruding from the cylinder body comprises fixation means to an external machine for driving a translation of the piston.

The kit of the invention designates an assembly of the elements. The kit is meant to be sterile and ready to use. It is a "single use kit", meaning that it will not undergo any cleaning operation in between batches or lots but will be discarded and replaced instead.

It is preferably provided in a package, ensuring sterile conditioning from the manufacturer site to the use site, like for example a sealed double bag.

The purpose of the kit is to avoid that the end user makes connections between the pump and the connecting lines in a grade A environment, which are sensitive manipulations introducing contamination risk. Moreover, these manipulations usually being made in an isolator, they are not only sensitive to contamination but also difficult to perform with gloves, and there is a risk that the connection is not properly done and fails during operation of the pump.

Preferably, the connecting line fastened to the inlet is equipped with an aseptic connector for connecting the line to the bulk tank. The aseptic connector allows the conservation of aseptic conditions of the filling circuit and protects it from foreign particles.

Preferably, the connecting line fastened to the outlet is equipped with an end-needle for dispensing liquid into the final containers. The end-needle is preferably inserted into a protective holster or case to avoid that the needle perforates the packaging and/or hurts the user.

In a preferred embodiment, the kit of the invention comprises several pumps, and as many inlet connecting lines and outlet connecting lines, with the inlet connecting lines being arranged downstream of a single main inlet line. In this configuration, several pumps arranged in parallel to be fed from a single supply tank. This means a main inlet line is divided into several inlet connecting lines, each connected to the inlet of a pump, each pump being equipped with an outlet connecting line. This arrangement allows parallel distribution in several containers, to speed up the process.

The pump of the kit comprises a cylinder body, meaning that the cylinder is closed at one end (with the exception of the inlet and the outlet). The other end is open to let the piston slide therein. The cylinder body has a cylinder axis.

In this case the pump is composed of a cylinder, hollowed out in a casing, and a straight piston sliding longitudinally with a predefined amplitude to define a metering chamber having a determined volume .

Contrary to the pumps described above regarding the prior art, the single action pump of the invention does not involve any rotation of the piston within the cylinder housing. The piston is only allowed reciprocal translation (sliding). This alleviates shear frictions that would be induced by rotation around the cylinder axis, which can be harmful for example when handling products comprising proteins. Preferably, the pump is devoid of a dynamic seal between the cylinder and the piston.

The inlet and the outlet are preferably arranged as nozzles protruding from the cylinder body, to allow threading on of the connecting lines. The nozzles have, for example, tubular section to allow for such connecting lines assembling.

In a preferred embodiment, the nozzles are arranged perpendicular to the axis of the cylinder. The nozzles can be comprised in the same plane, which is perpendicular to the cylinder axis. The nozzles can be aligned or can be arranged with an angle between them, for example an angle of 90°.

When the nozzles are in the same plane, this implies that the connecting lines extend perpendicularly from the cylinder body. They will thus be horizontal when the pump is operated in a vertical position.

The connecting lines in the kit of the invention are made with a flexible material allowing the line to be obturated upon pinching. This means that the pump kit of the invention is designed to be operated by alternatively hindering fluid displacement in the connecting line fastened to the inlet (inlet line) and in the connecting line fastened to the outlet (outlet line). This implies that the kit is devoid of check valves or non-return valves for operating the pump. The inlet and the outlet arranged in the cylinder body are mere openings, devoid of any valve. This seriously lowers the risk of dysfunction of the pump which could result from blocking of the valve, due for example to high viscosity or crystallization or damage of valve elements.

The connecting lines are typically silicone-based tubes or hoses.

In a preferred embodiment, the cylinder body comprises at least two assembled sections, which can be made of different materials. The two sections are typically a cylinder head wherein the inlet and outlet are arranged, and a piston housing, wherein the piston can slide, maintained together by joining means.

The joining means can be sanitary fitting, to maintain proper pump sealing during manipulation and operation of the pump. Sanitary fittings are well known to the person skilled in the art. Such fitting can comprise a joint to be placed in between the two parts to connect and a clamp to maintain sections assembled. This may imply that the sections have a specific design incorporating a rim at the connection place. There can also be other types of joining means.

In a preferred embodiment, the piston and the cylinder housing (at least the piston housing part of the cylinder body) are made of ceramic.

When the cylinder body comprises several sections, the cylinder head can be made in a plastic material, like for example polypropylene. The joining means can also be made of polypropylene, or in any other suitable material.

Pinching is here equivalent to squeezing or obturating by applying a pressure on said line.

The pinching means can be any means known to the person skilled in the art for obturating tubes or hoses.

The driving system of the invention can be a single apparatus or machine or can be different machines connected to a synchronization module.

The driving system of the invention can further comprise fixing means to secure the pump in a fixed position during operations. These fixing means comprise at least means to immobilize the cylinder body of the pump.

Advantageously, engaging the pump within the fixation means allows simultaneous engagement or positioning of the fixation means of the piston with regards to the driving arm. This can be completed by self-interlocking means or by actioning further piston locking means.

In a particular embodiment of the invention, the fixing means are arranged to allow insertion of the cylinder body by translation along the axis of the cylinder body but not by translation perpendicularly to the axis of the cylinder body. As the pump is usually meant to be positioned with the axis of the cylindrical body positioned vertically, such a configuration allows to insert the pump in the fixing means via:
- positioning the pump slightly above its operating position, so as to position the inlet and outlet connecting lines above the pinching valves and
- translating the pump down inside the fixation means with a vertical movement, to engage the inlet and outlet connecting lines in their respective pinching valve elements.

The pump cannot be further pulled forward.

Abutment means can be further foreseen to position the pump at a predefined height. Further securing means can also be foreseen to enhance locking of the pump in operating position.

This configuration presents several advantages.

First, it allows the use of a simple gripper to position the pump into operating configuration. Grippers are known in the field of pumps, as they allow to maintain assembled the piston within the cylinder body. The gripper can be a simple clamp which will release the pump upon lateral pulling. The pump not being able to move laterally, the operation is then very simple.

A second advantage is that it allows to position the connecting lines inside the pinch valves prior to fixing the pump. As the connecting lines have a degree of flexibility, they can absorb the stress applied upon them during the vertical movement of the pump for fixing, especially as in this step, the pinch valves are both open. Positioning the connecting lines is also made easy by using a gripper.

In a particular embodiment, particularly suited for the placement and operation of the pump kit of the invention, a pinch valve comprises a fixed vertical cylindrical pin and a mobile vertical cylindrical pin, the mobile vertical pin being arranged to move along a circle arc towards the fixed pin such that a flexible line (the connecting line of the kit of the invention) positioned in between both pins is pinched to full obturation, and away from the fixed pin to unobturate the flexible line. This design, i.e. the fact the pins are cylindrical, and that the mobile pin moves along a circle arc, presents the advantage of reducing the stress applied to tubings and reduce the risk of spallation effect.

The invention will be better understood with the detailed description below referring to the drawing in which:
Figure 1 is a schematic view of the kit of the invention;
Figure 2 is an exploded view in perspective of a pump for a kit of the invention;
Figure 3 is an assembled view in perspective of the pump of figure 2;
Figure 4 is a cut view of the pump of figure 2 and 3;
Figure 5 illustrates in perspective the pump portion of the kit of the invention in operating position within a driving system of the invention;
Figure 6 is a perspective view of a pump of a kit of the invention hold by a gripper;

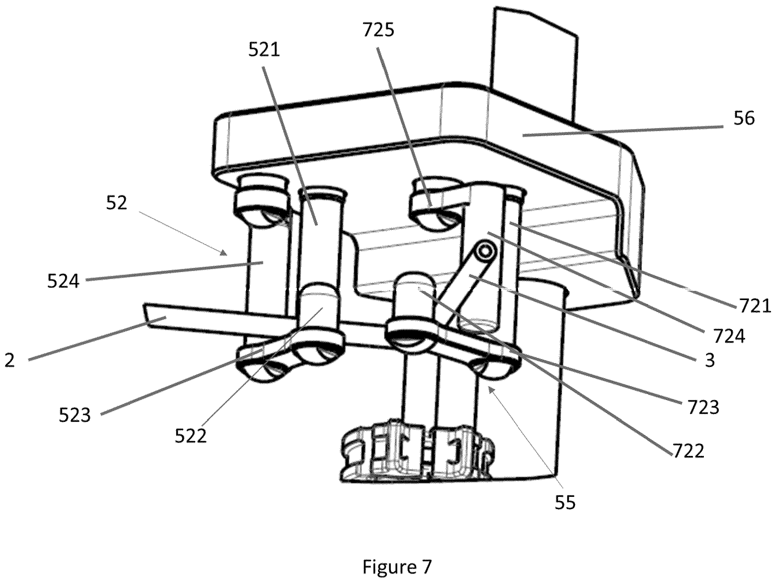

Figure 7 is an enlarged view of the pinch valves of the system of figure 5, and
Figure 8 is a schematic view of a kit of the invention comprising multiple pumps.

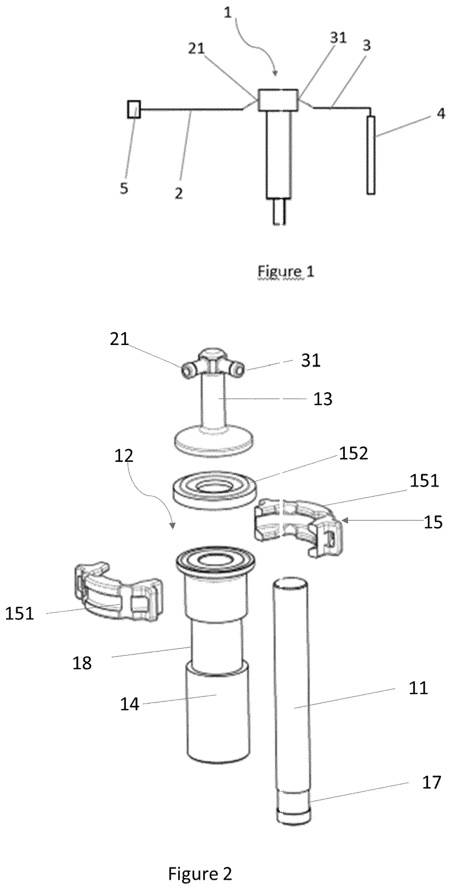

With reference to figure 1, a kit of the invention comprises a single action pump 1, an inlet connecting line 2 fastened to an inlet 21 of the pump and an outlet connecting line 3 fastened to the outlet 31 of the pump. The connecting lines 2 and 3 are tubes made with a flexible material, here for example pharmaceutical grade silicone, allowing the line to be obturated upon pinching. The outlet connecting line 3 here is equipped with an end-needle 4 for dispensing liquid into the final containers. This end-needle is preferably inserted into a protective holster. An aseptic connector 5 is here also foreseen at the extremity of the inlet connecting line.

The needle is here illustrated as example, the outlet connecting line could end with another distribution means or a connector to distribution means installed at the operating facilities.

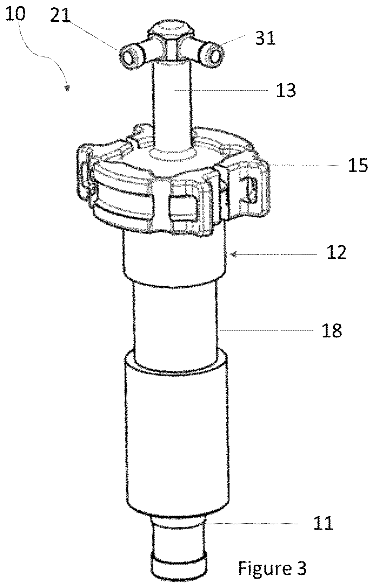

With reference to figures 2 to 3, adhering to the same numbering for similar elements, the pump 10 of the kit has a piston 11 slidingly arranged within a cylinder body 12.

The cylinder body 12 comprises here two assembled sections: a cylinder head 13 wherein the fluid inlet 21 and fluid outlet 31 are arranged, and a piston housing 14, wherein the piston can slide. The piston housing 14 has a larger diameter than the cylinder head 13, the piston can therefore not penetrate into the cylinder head. A sanitary fitting 15, comprising a clamp 151 and a joint 152 secured together with a zip tie (not represented) maintains both parts of the pump together.

The pump is here made of two materials: the piston 11 and the piston housing are made of ceramic, while the cylinder head is made of polypropylene.

The cylinder body has an axis AA'. The inlet 21 and an outlet 31 both allowing fluid communication between the outside of the cylinder and a metering chamber 16 inside of the cylinder body. The metering chamber 16 is here further defined by the top extremity of the piston 11 in the piston housing.

The inlet nozzle 21 and an outlet nozzle 31 are here arranged in plane perpendicular to the AA' axis, with an angle of about 90° between them.

The lower extremity of the piston 11 is protruding from the cylinder 14 and comprises fixation means to an external machine for driving a translation of the piston, these fixation means are here an annular recess 17 towards the end of the piston.

The piston housing 14 has here an annular recess 18, over a portion of its height, towards the middle. This recess can be useful for handling and fixing the pump in operating position on a driving system.

With such a design, the pump of the invention can handle very small volumes of liquid. Indeed, the volume of liquid distributed at each stroke of the piston is determined by the amplitude of the translation of the piston within the piston housing. The pump can be designed with very small internal diameters allowing to distribute less than 0.1 mL, like for example 0.08 mL. The absence of check valves in the system is important, as such valves do not allow stable accuracy with such small volumes.

Though the kit of the invention is suitable to be installed on known driving systems equipped with pinched valves, the applicant has additionally designed a specific system which includes new pinching means.

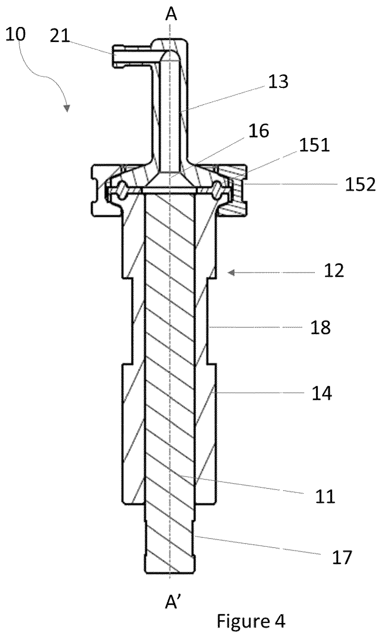

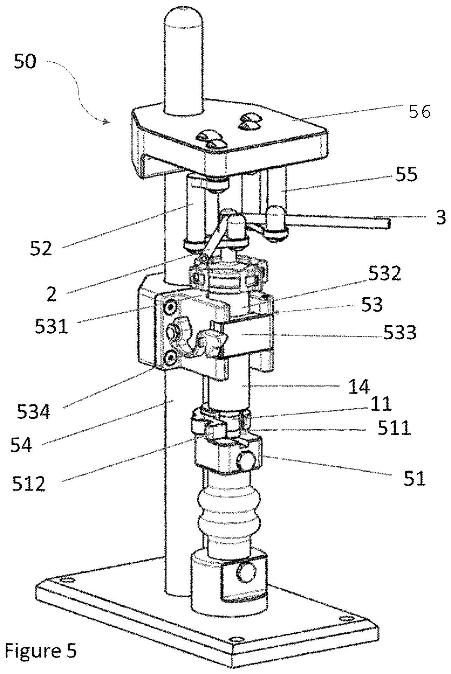

With reference to figure 5, the kit of figures 1 to 4 is installed on a driving system 50 adapted to operate the kit of the invention, comprising a mobile arm 51 arranged to drive a reciprocal translation of the piston 11 within the piston housing of the cylinder body, and pinching means arranged to alternatively pinch the inlet and outlet connecting lines 2 and 3.

The driving system 50 further comprises fixing means 53 to secure the pump in a fixed position during operations. These fixing means here comprise a holding section 531, fixed on a stand 54. The holding section has a hollow cylinder part for receiving or lodging the cylindrical body of the pump, the AA' axis of the pumps is meant to align with the axis of the hollow cylinder. This hollow cylinder has a radial opening 532 to allow axial insertion of the pump. Radial opening 532 is designed to prevent lateral exit of the pump on it is placed. A hinged door 533 can obturate, at least partially, the radial opening thereby preventing axial exit of the pump from its lodging. A rotating latch 534 is further foreseen to secure the door 533 in a closed position.

The piston 11 is also secured to the driving arm. The extremity of the driving arm 51 also comprises a cylindrical lodging with a radial opening 511 in which the extremity of the piston is inserted. A rotating lock 512 can be actioned to obturate the radial opening 511. The rotating lock is shaped to be complementary to the recess 17 in the piston to work as abutment means in locked position and thereby prevent unwanted vertical movement of the piston relative to the arm. This ensures optimal precision of volumetric delivery.

Two pinching systems 52 and 55 are arranged in the driving above the means to fix the pump, underneath a platform 56 here also fixed on stand 54.

The pinch valve 52 is here for managing fluid circulation in the inlet line 2. It comprises a fixed vertical pillar 521 here hanging from platform 56 and a mobile vertical pin 522, the mobile vertical pin is here an upwards ergot 522 lying at the extremity of a horizontal segment 523. The other extremity of the horizontal segment is fixed to a pivot axis 524 also hanging from platform 56 connected to a motor (not represented). Pivoting axis 524 will drive a rotation of the squeezing pin 522 along a circle arc towards the fixed pillar 521, thereby obturating the line. The distance between the pivot axis 524 and pin 522 on the segment 523 when the valve is open is such that the inlet connecting line can rest in between on the segment.

On the same principle, but with a slightly different design, the pinch valve 55 is here for managing fluid circulation in the outlet line 3. It comprises a fixed vertical pillar 721 here hanging from platform 56 at the bottom of with extends a horizontal segment 723, at the end of which lies an upwards ergot 722. A mobile vertical pillar 724 is hanging from platform 56 in a deported manner around a pivot 725 connected to a motor (not represented). Pivoting 725 will drive a rotation of the squeezing pillar 724 along a circle arc towards the fixed ergot 722, thereby obturating the line 3. The distance between the pillar 721 and ergot 722 on the segment 723 when the valve is open is such that the outlet connecting line can rest in between on the segment. Though the pinch valves 52 and 55 are here not identical, and have a specific design, the person skilled in the art will understand that they could be similar, or inverted or any other suitable pinch valve could be used. One advantage of this specific design is that the connecting lines are supported by gravity, without any further securing operation, thereby facilitating mounting operations.

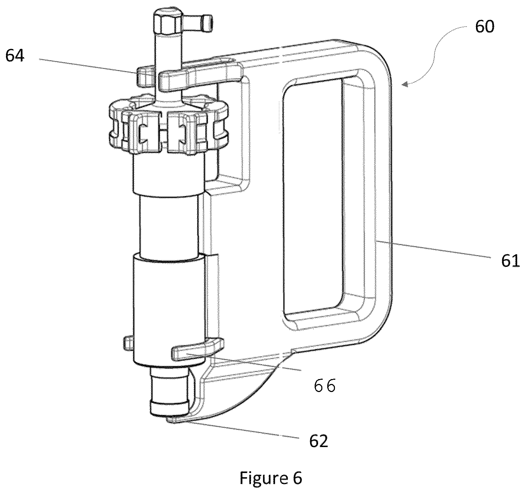

To place the pump in operating position, a gripper 60 can be used as illustrated on figure 6. The gripper 60 is a conventional gripper and comprises a handle 61, a clamp 64 to grab the cylinder head of the pump, a clamp 66 to grab the piston housing and a spigot 62 to prevent the piston to move outwards of the piston housing.

The connecting lines of the pump are not illustrated on figures 2 to 6 for clarity reasons but come fixed on the pump in the kit.

The operator takes the pump with the connecting lines 2 and 3, maintaining it in a general vertical position. He moves the pump towards the driving system, passing the head of the pump in between the two pinch valves 52, until the connecting lines 2 and 3 are each above a horizontal segment 523 of the valves. This movement also allow to insert the cylinder body of the pump in the holding section 531 of the driving system, and to place the extremity of piston 11 partially in or just above its lodging in the driving arm.

The operator applies a vertical translation downwards until the cylinder body 12 rests on a flat surface inside the radial opening 532 of cylinder holding attachment 53. This also allows the extremity of piston 11 to be fully inserted in its lodging in the driving arm. The operator can rotate lock 512 to fasten piston 11 to the arm 51. With the pump laterally secured, the gripper can be removed by the operator with a horizontal pulling movement. The operator can finally close door 533 and lock it by rotating the latch 534. Advantageously, the door 533 has a shape complementary to recess 18 in the cylinder body of the pump to also vertically locks the position of the pump body.

The operator then only needs to place the needle end of the outlet line 3 where it should be and connect inlet line 2 to the reservoir of product to distribute. The pump is ready to operate.

Both the mobile arm and the pinching means are motorized means. They need to operate in a synchronized manner as known to a person skilled in the art. When the piston is translated outwards of the cylinder body, the pinch valve around outlet connecting valve 3 is pressing on the line to obturate it, while the pinch valve around inlet connecting valve 2 is open, to allow liquid from reservoir to enter the pump. When the piston is translated upwards of the cylinder body, the pinch valve around outlet connecting valve 3 is open, while the pinch valve around inlet connecting valve 2 is pressing on the line to obturate it, to allow liquid flow out of the pump towards the distributing needle.

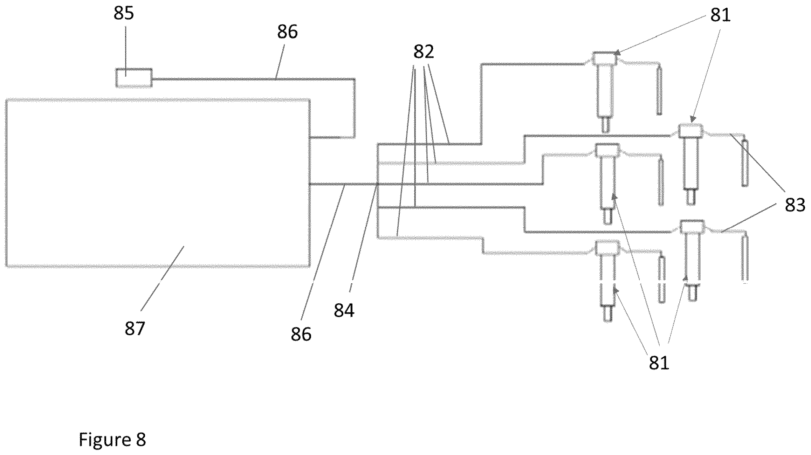

With reference to figure 8, a kit of the invention can also comprise several pumps in parallel. For example, five single action pumps 81 have each an inlet connecting line 82 fastened to an inlet of the pump and an outlet connecting line 33 fastened to the outlet of the pump. The inlet connecting lines 82 are merged into a single line 86 upstream of the pumps at point 84.

An aseptic connector 85 is here also foreseen at the extremity of the inlet connecting line. A surge bag 87 is here additionally inserted on the single line 86.

## Claims

1. A set comprising a kit and a driving system adapted to operate the kit, said kit comprising:
- a pump (1;10) with a piston (11) slidingly arranged within a cylinder body (12), the cylinder body being arranged with an inlet (21) and an outlet (31) both allowing fluid communication between the outside of the cylinder and a metering chamber (16) inside of the cylinder body, said metering chamber being further defined by one extremity of the piston lying within the cylinder body and wherein the other extremity of the piston is protruding from the cylinder body;
- an inlet connecting line (2) fastened to the inlet;
- an outlet connecting line (3) fastened to the outlet;
wherein the connecting lines are made with a flexible material allowing the line to be obturated upon pinching;
- said driving system (50) comprising pinching means (55) arranged for pinching the outlet connecting line and for managing a fluid circulation in the outlet connecting line, said driving system further comprising a mobile arm (51) arranged to hold said other extremity of the piston and for driving a reciprocal translation of said piston within the cylinder body,
**characterized in that** the pinching means (52) are further provided for pinching the inlet connecting line and for managing a fluid circulation in the inlet connecting line, said pinching means being further arranged to alternatively pinch the inlet connecting line and the outlet connecting line, said mobile arm and said pinching means being synchronized so as to close the output connecting line when the piston is translated outwards of the cylinder body and to close the input connecting line when the piston is translated inwards of the cylinder body.

2. The set according to claim 1, wherein the outlet connecting line is equipped with an end-needle (4), preferably inserted into a protective holster.

3. The set according to one of claims 1 or 2, sterilized and further comprising a sterile packaging in which it is inserted.

4. The set according to one of claims 1 to 3, wherein the inlet connecting line is equipped with an aseptic connector for connecting the line to a bulk tank.

5. The set according to one of claims 1 to 4, wherein the inlet and the outlet are arranged with nozzles protruding from the cylinder body.

6. The set according to claim 5, wherein the nozzles are arranged perpendicular to the axis of the cylinder, preferably in the same plane.

7. The set according to one of claims 1 to 6, wherein the cylinder body of the pump comprises at least two assembled sections.

8. The set according to claim 7, wherein the two sections are a cylinder head wherein the inlet and outlet are arranged, and a piston housing, wherein the piston can slide, both sections being maintained together by joining means.

9. The set according to claim 8 wherein the joining means are a sanitary fitting.

10. The set according to one of claims 1 to 9, wherein the piston and at least part of the cylinder housing are made of ceramic.

11. The set according to one of claims 1 to 10, comprises several pumps, each equipped with an inlet connecting line and an outlet connecting line, and wherein the inlet connecting lines are arranged downstream of a single main inlet line.

12. The set according to anyone of the claims 1 to 11, further comprising fixing means to secure the pump in a fixed position during operations.

13. The set according to claim 12, wherein the fixing means are arranged to allow fixation upon downwards translation of the cylinder body along the axis of the cylinder body.

14. The set according to anyone of claims 1 to 13, further comprising locking means of the piston of the pump to the mobile arm.

15. The set according to one of claims 1 to 14, wherein the driving system comprises a pinch valve having a fixed vertical cylindrical pin and a mobile vertical cylindrical pin, the mobile vertical pin being arranged to move along a circle arc towards the fixed pin such that a flexible line positioned in between both pins is pinched to full obturation, and away from the fixed pin to unobturate the flexible line.

## Patentansprüche

1. Bausatz, der einen Kit und einen Antriebssystem zum Betrieb des Kits umfasst, wobei das Kit Folgendes umfasst:
- eine Pumpe (1; 109) mit einem Kolben (11), der gleitend innerhalb einem Zylinderkörper (12) angeordnet ist, wobei der Zylinderkörper mit einem Einlass (21) und einem Auslass (31) versehen ist, die beide eine fließende Verbindung zwischen der Außenseite des Zylinders und einer Dosierkammer (16) innerhalb des Zylinderkörpers ermöglichen, wobei genannte Dosierkammer ferner dadurch definiert ist, dass sich ein Ende des Kolbens im Zylinderkörper befindet und das andere Ende des Kolbens aus dem Zylinderkörper herausragt;
- eine am Einlass befestigte Einlassverbindungsleitung (2);
- eine an den Auslass befestigte Auslassverbindungsleitung (3);
wobei die Verbindungsleitungen aus einem flexiblen Material hergestellt sind, das es ermöglicht, die Leitung durch Zusammendrücken zu verschließen;
- wobei das Antriebsystem (50) Klemmmittelen (55) umfasst, die zum Klemmen der Auslassverbindungsleitung und zum Steuern eines Flüssigkeitsumlaufs in der Auslassverbindungsleitung angeordnet ist, und das Antriebssystem ferner einen beweglichen Arm (51) umfasst, der so angeordnet ist, dass er das andere Ende des Kolbens hält und eine Hin- und Herbewegung genanntes Kolbens im Zylinderkörper bewirkt,
**dadurch gekennzeichnet, dass** die Klemmmittelen (52) ferner zum Klemmen der Einlassverbindungsleitung und zum Steuern eines Flüssigkeitsumlaufs in der Einlassverbindungsleitung vorgesehen ist, wobei die Klemmmittelen ferner so angeordnet ist, dass sie abwechselnd die Einlassverbindungsleitung und die Auslassverbindungsleitung abklemmt, und genannter bewegliche Arm und die Klemmmittelen so synchronisiert sind, dass sie die Auslassverbindungsleitung schließen, wenn der Kolben aus dem Zylinderkörper herausgeschoben wird, und die Einlassverbindungsleitung schließen, wenn der Kolben innerhalb den Zylinderkörper hineingeschoben wird.

2. Bausatz nach Anspruch 1, wobei die Auslassverbindungsleitung mit einer Endnadel (4) ausgestattet ist, die vorzugsweise in eine Schutzhülle eingeführt ist.

3. Bausatz nach einem der Ansprüche 1 oder 2, sterilisiert und darüber hinaus eine sterile Verpackung umfasst, in die er eingeführt ist.

4. Bausatz nach einem der Ansprüche 1 bis 3, wobei die Einlassverbindungsleitung mit einem aseptischen Anschlussstück zum Verbinden der Leitung mit einem Sammelbehälter ausgestattet ist.

5. Bausatz nach einem der Ansprüche 1 bis 4, wobei der Einlass und der Auslass mit Düsen angeordnet sind, die aus dem Zylinderkörper herausragen.

6. Bausatz nach Anspruch 5, wobei die Düsen senkrecht zur Achse des Zylinders angeordnet sind, vorzugsweise in derselben Ebene.

7. Bausatz nach einem der Ansprüche 1 bis 6, wobei der Zylinderkörper der Pumpe mindestens zwei zusammengefügte Abschnitte umfasst.

8. Bausatz nach Anspruch 7, wobei die beiden Abschnitte einem Zylinderkopf sind, in dem der Einlass und der Auslass angeordnet sind, und einem Kolbengehäuse, in dem sich der Kolben verschieben kann, wobei beide Abschnitte durch Verbindungsmittel zusammengehalten werden.

9. Bausatz nach Anspruch 8, wobei die Verbindungsmittel sanitärtechnische Fittings sind.

10. Bausatz nach einem der Ansprüche 1 bis 9, wobei der Kolben und zumindest ein Teil des Zylindergehäuses aus Keramik hergestellt sind.

11. Bausatz nach einem der Ansprüche 1 bis 10, wobei dieser mehrere Pumpen umfasst, die jeweils mit einer Einlassverbindungsleitung und einer Auslassverbindungsleitung ausgestattet sind, und die Einlassverbindungs-leitungen nach einer einzigen Haupteinlassleitung angeordnet sind.

12. Bausatz nach einem der Ansprüche 1 bis 11, der ferner Befestigungsmittel umfasst, um die Pumpe während des Betriebs in einer festen Position zu halten.

13. Bausatz nach Anspruch 12, wobei die Befestigungsmittel so angeordnet sind, dass sie eine Befestigung bei einer Abwärtsbewegung des Zylinderkörpers entlang der Achse des Zylinderkörpers ermöglichen.

14. Bausatz nach einem der Ansprüche 1 bis 13, der ferner Verriegelungsmittel des Pumpenkolbens am beweglichen Arm umfasst.

15. Bausatz nach einem der Ansprüche 1 bis 14, wobei das Antriebssystem ein Quetschventil mit einem feststehenden vertikalen Zylinderstift und einem beweglichen vertikalen Zylinderstift umfasst, und der bewegliche vertikale Stift so angeordnet ist, dass er sich entlang eines Kreisbogens in Richtung des festen Stifts bewegt, sodass eine zwischen beiden Stiften angeordnete flexible Leitung vollständig verschlossen wird, und sich vom festen Stift wegbewegt, um die flexible Leitung zu öffnen.

## Revendications

1. Ensemble comprenant un kit et un système d'entraînement adapté à faire fonctionner le kit, ledit kit comprenant :
- une pompe (1 ;10) avec un piston (11) agencé de manière coulissante à l'intérieur d'un corps de cylindre (12), le corps de cylindre étant agencé avec une entrée (21) et une sortie (31) permettant toutes deux la communication de fluide entre l'extérieur du cylindre et une chambre de dosage (16) à l'intérieur du corps de cylindre, ladite chambre de dosage étant en outre définie par une extrémité du piston se trouvant à l'intérieur du corps de cylindre et l'autre extrémité du piston faisant saillie à partir du corps de cylindre ;
- une ligne de raccordement d'entrée (2) fixée à l'entrée ;
- une ligne de raccordement de sortie (3) fixée à la sortie ; dans lequel les lignes de raccordement sont réalisées avec un matériau flexible permettant d'obturer la ligne lors du pincement ;
- ledit système d'entraînement (50) comprenant des moyens de pincement (55) agencés pour pincer la ligne de raccordement de sortie et pour gérer une circulation de fluide dans la ligne de raccordement de sortie, ledit système d'entraînement comprenant en outre un bras mobile (51) agencé pour retenir ladite autre extrémité du piston et pour entraîner une translation de va-et-vient dudit piston à l'intérieur du corps de cylindre,
**caractérisé en ce que** les moyens de pincement (52) sont en outre prévus pour pincer la ligne de raccordement d'entrée et pour gérer une circulation de fluide dans la ligne de raccordement d'entrée, lesdits moyens de pincement étant en outre agencés pour pincer alternativement la ligne de raccordement d'entrée et la ligne de raccordement de sortie, ledit bras mobile et lesdits moyens de pincement étant synchronisés de manière à fermer la ligne de raccordement de sortie lorsque le piston est translaté vers l'extérieur du corps de cylindre et à fermer la ligne de raccordement d'entrée lorsque le piston est translaté vers l'intérieur du corps de cylindre.

2. Ensemble selon la revendication 1, dans lequel la ligne de raccordement de sortie est équipée d'une aiguille d'extrémité (4), de préférence insérée dans un étui de protection.

3. Ensemble selon l'une des revendications 1 ou 2, stérilisé et comprenant en outre un emballage stérile dans lequel il est inséré.

4. Ensemble selon l'une des revendications 1 à 3, dans lequel la ligne de raccordement d'entrée est équipée d'un connecteur aseptique pour raccorder la ligne à un réservoir de vrac.

5. Ensemble selon l'une des revendications 1 à 4, dans lequel l'entrée et la sortie sont agencées avec des buses faisant saillie à partir du corps de cylindre.

6. Ensemble selon la revendication 5, dans lequel les buses sont agencées perpendiculairement à l'axe du cylindre, de préférence dans le même plan.

7. Ensemble selon l'une des revendications 1 à 6, dans lequel le corps de cylindre de la pompe comprend au moins deux sections assemblées.

8. Ensemble selon la revendication 7, dans lequel les deux sections sont une tête de cylindre où l'entrée et la sortie sont agencées, et un logement de piston où le piston peut coulisser, les deux sections étant maintenues ensemble par des moyens de jonction.

9. Ensemble selon la revendication 8, dans lequel les moyens de jonction sont un raccord sanitaire.

10. Ensemble selon l'une des revendications 1 à 9, dans lequel le piston et au moins une partie du logement de cylindre sont réalisés en céramique.

11. Ensemble selon l'une des revendications 1 à 10, comprenant plusieurs pompes, chacune étant équipée d'une ligne de raccordement d'entrée et d'une ligne de raccordement de sortie, et dans lequel les lignes de raccordement d'entrée sont agencées en aval d'une ligne d'entrée principale unique.

12. Ensemble selon l'une quelconque des revendications 1 à 11, comprenant en outre des moyens de fixation pour fixer la pompe dans une position fixe pendant le fonctionnement.

13. Ensemble selon la revendication 12, dans lequel les moyens de fixation sont agencés pour permettre la fixation lors de la translation vers le bas du corps de cylindre le long de l'axe du corps de cylindre.

14. Ensemble selon l'une quelconque des revendications 1 à 13, comprenant en outre des moyens de verrouillage du piston de la pompe sur le bras mobile.

15. Ensemble selon l'une des revendications 1 à 14, dans lequel le système d'entraînement comprend une soupape à pincement présentant une goupille cylindrique verticale fixe et une goupille cylindrique verticale mobile, la goupille verticale mobile étant agencée pour se déplacer le long d'un arc de cercle vers la goupille fixe de telle sorte qu'une ligne flexible positionnée entre les deux goupilles soit pincée jusqu'à obturation complète, et à distance de la goupille fixe pour désobturer la ligne flexible.
